(19) Europäisches Patentamt
European Patent Office
Office européen des brevets

(11) **EP 3 286 306 B1**

(12) **EUROPEAN PATENT SPECIFICATION**

(45) Date of publication and mention
of the grant of the patent:
**09.12.2020 Bulletin 2020/50**

(51) Int Cl.:
*C12N 9/32* (2006.01)    *A21D 2/18* (2006.01)
*A21D 2/40* (2006.01)    *A21D 6/00* (2006.01)
*A21D 8/04* (2006.01)    *C08B 30/18* (2006.01)
*C12N 9/26* (2006.01)    *C13K 1/06* (2006.01)

(21) Application number: **16783825.9**

(22) Date of filing: **21.04.2016**

(86) International application number:
**PCT/US2016/028574**

(87) International publication number:
**WO 2016/172298 (27.10.2016 Gazette 2016/43)**

(54) **NOVEL PROCESS**

NEUARTIGES VERFAHREN

NOUVEAU PROCÉDÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.04.2015 US 201562150970 P
14.10.2015 US 201562241314 P**

(43) Date of publication of application:
**28.02.2018 Bulletin 2018/09**

(73) Proprietors:
• **GlaxoSmithKline Consumer Healthcare Holdings
(US)
LLC
Wilmington, DE 19808 (US)**
• **GlaxoSmithKline Consumer Healthcare (UK)
IP Limited
Middlesex TW8 9GS (GB)**

(72) Inventors:
• **JONES, John, Benjamin
Slough
Berkshire SL1 3NW (GB)**
• **FINLAY, Richard, Matthew John
Slough
Berkshire SL1 3NW (GB)**
• **HEATH, Paul, John
Slough
Berkshire SL1 3NW (GB)**
• **LODAYA, Mayur, P.
Research Triangle Park, North Carolina 27709
(US)**

(74) Representative: **Warner, Guy Jonathan
Unilever PLC
Unilever Patent Group
Bronland 14
6708 WH Wageningen (NL)**

(56) References cited:
US-A- 3 137 592        US-A1- 2002 022 076
US-A1- 2006 246 192    US-A1- 2013 071 491
US-B1- 6 190 591

• GOVINDASAMY ET AL: "High moisture twin
screw extrusion of sago starch. II.
Saccharification as influenced by
thermomechanical history", CARBOHYDRATE
POLYM, APPLIED SCIENCE PUBLISHERS, LTD.
BARKING, GB, vol. 32, no. 3-4, 1 March 1997
(1997-03-01), pages 267-274, XP005876495, ISSN:
0144-8617, DOI: 10.1016/S0144-8617(96)00163-4
• ROUSSEL L ET AL: "SEQUENTIAL HEAT
GELATINIZATION AND ENZYMATIC
HYDROLYSIS OF CORN STARCH IN AN
EXTRUSION REACTOR. OPTIMIZATION FOR A
MAXIMUM DEXTROSE EQUIVALENT", LWT-
FOOD SCIENCE AND TECHNO, ACADEMIC
PRESS, UNITED KINGDOM, vol. 24, 1 January
1991 (1991-01-01), pages 449-458, XP008074609,
ISSN: 0023-6438
• VASANTHAN, T ET AL.: 'Dextrinization of Starch
in Barley Flours with Thermostable
alpha-Amylase by Extrusion Cooking.' STARCH.
vol. 53, no. 12, December 2001, pages 616 - 622,
XP001110714

EP 3 286 306 B1

**Description**

**RELATED APPLICATIONS**

This application claims the benefit of US Provisional 62/150970 filed 22 April 2015 and US Provisional 62/241314 filed 14 October 2015.

**FIELD OF THE INVENTION**

The present invention relates to the use of extrusion technology in a process for the low-moisture continuous gelatinisation and the enzymatic hydrolysis of flour to provide a dextrinised product having a dextrose equivalent (DE) of at least 15 and to a process for drying a dextrinised product.

**BACKGROUND OF THE INVENTION**

The conversion of insoluble granular starch found in flour to glucose, maltose and/or other soluble dextrins is an important process to obtain products, such as speciality syrups and sugars. Solubilisation of starch granules in water requires heat and time to disrupt the crystalline structure. The more water used to solubilise the granules, the more energy is required to heat the water. If high-solids syrup or dried and powdered forms of the hydrolysed starch product are required then there is an energy benefit to reducing the total quantity of water required to complete the gelatinisation and hydrolysis and subsequently evaporate excess water.

Methods for dextrinisation of starch are known, such as acid or enzymatic hydrolysis of pregelatinised starch. Vasanthan *et al* 2001, describes a process for dextrinisation of starch in barley flours with thermostable $\alpha$-amylase by extrusion cooking. (Dextrinisation of Starch in Barley Flours with Thermostable $\alpha$-amylase by Extrusion Cooking, Thava Vasanthan, Judy Yeung, Ratnajothi Hoover; Starch/Stärke, 53 (2001), 616-622*).*

Barley grains from two hull-less varieties, Phoenix and CDC-Candle, were extruded in a twin-screw extruder at 90-140°C, 20-50% moisture, and 0-4% $\alpha$-amylase concentration. The effects of extrusion conditions on the degree of hydrolysis, dextrose equivalent and mono/di/oligosaccharide composition of the dextrinised flour were determined. The data showed that the degree of hydrolysis and dextrose equivalent increased with increasing $\alpha$-amylase concentration and moisture level. The optimum processing temperature was 100°C to maximise $\alpha$-amylase activity while minimizing its inactivation. At an enzyme level of 2% starch basis, temperature of 100°C, 50 RPM and moisture content of 35%, the dextrose equivalent (DE) of Phoenix variety was 14.6 $\pm$ 0.2. For CDC-candle the DE was 13.2 $\pm$ 0.6. At an enzyme concentration of 4% starch basis, the DE was 24.3 $\pm$ 1.4 for Phoenix and 21.0 $\pm$ 1.0 for CDC-Candle. Govindasamy et al (Carbohydrate Polymers 32(1997) 267-274) discloses a process for gelatinisation and saccharification of sago starch, comprising passing said starch through a twin-screw extruder using amyloglucosidase (y-amylase). Roussel et al (Lebensm.-Wiss. U.- Technol. 24, 449-458 (1991)) discloses a method for gelatinisation and saccharification of corn starch comprising extruding corn starch at a moisture level of 40% at a barrel temperature for liquefaction of 105°C and a screw speed of 140 rpm in combination with alpha-amylase. Vasanthan et al (Starch/Stärke 53(2001), 616-622) describes the dextrinization of barley flour using a twin-screw extruder at a low screw speed of 50 rpm at three different enzyme concentrations of 0, 2 and4% by weight, four different temperatures of 90, 100, 120 and 140C and three different moisture levels of 20, 35 and 50% as described in section 2.3 on page 617 of this document.

The present invention is based on the discovery that extrusion gelatinisation and enzymatic hydrolysis of starch in flour can be increased by the use of high shear even though the residence time is decreased. Contrary to the above noted paper a dextrinised product having a dextrose equivalent of at least 15 may be obtained using low enzyme concentrations and low moisture contents.

Further the present inventors have found that a dextrinised product may be dried in an extruder.

**SUMMARY OF THE INVENTION**

According to the present invention there is provided a process for gelatinisation and the enzymatic hydrolysis of flour which comprises passing said flour through a twin-screw extruder in the presence of 0.025 % w/w to 1.5% w/w relative to flour of an $\alpha$-amylase hydrolytic enzyme and in the presence of water to provide a moisture level from 30% to 45% w/w relative to the total quantity of ingredients, at a screw speed from about 300 to about 800 Revolutions Per Minute, (RPM), and a temperature in the range from about 80°C to about 160°C; the dimensions of the extruder being such that the residence time of the flour through the extruder is from 60 to 120 seconds and results in a dextrinised product having a dextrose equivalent of at least 15.

The advantage of the process of the present invention is the rapid production of a dextrinised product with low levels of enzyme and water addition. The overall result is a more efficient process than a traditional saccharification/evaporation process.

In another aspect of the present invention there is provided a process for drying a dextrinised product which comprises passing the said dextrinised product through a twin-screw extruder having at least one vent to allow the moisture that is released in the form of water vapour to be removed. The invention is defined by the claims.

## BRIEF DESCRIPTION OF THE DRAWINGS

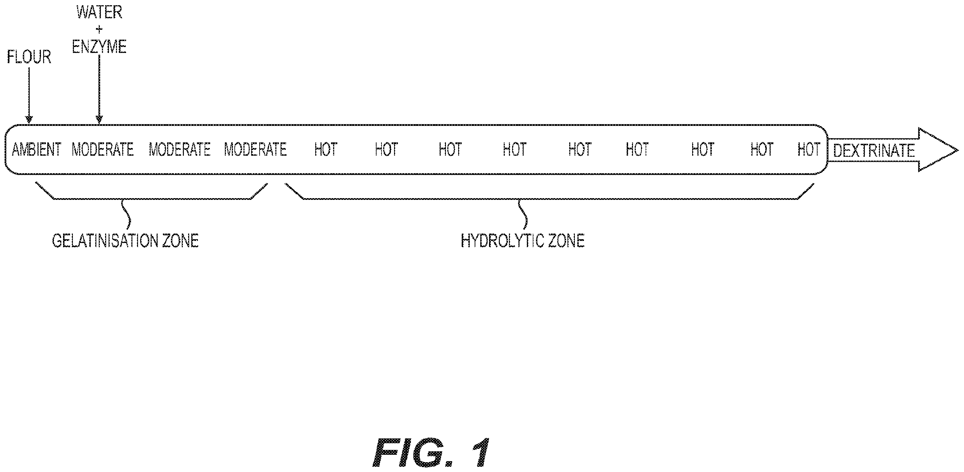

Figure 1. Is an example of a typical extruder configuration for gelatinisation and enzymatic hydrolysis.

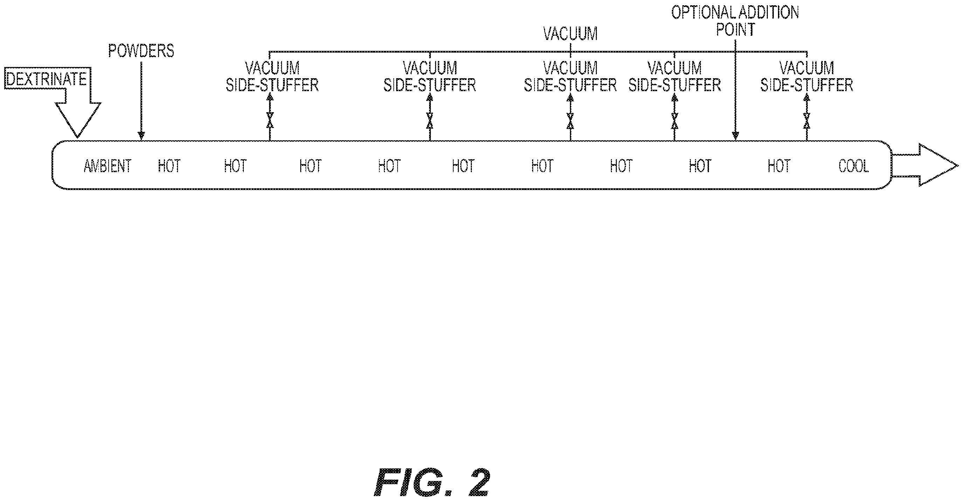

Figure 2. Is an example of a typical extruder configuration for drying of a dextrinised product.

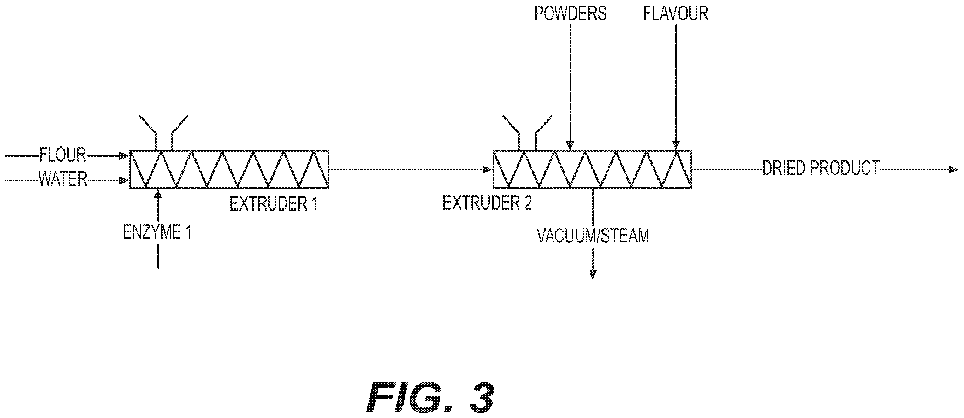

Figure 3. Is an example of two extruders connected in series, for the purpose of continuous gelatinisation, hydrolysis, formulation assembly and drying.

Definitions:

Dextrose Equivalent (DE):

This is an industry standard way of expressing the total concentration of reducing sugars and is expressed relative to D-glucose on a dry weight basis. Unhydrolysed granular starch has a DE of 0 and anhydrous D-glucose has a DE of 100.

Gelatinisation:

Starch gelatinisation is the process during which the crystalline structure of the starch granule is disrupted by heat and water resulting in water uptake, swelling of granules, crystalline melting, loss of birefringence and starch solubilisation.

Hydrolysis:

Means the cleavage of chemical bonds by the addition of water.

Dextrinisation:

The hydrolysis reaction applied to carbohydrate polymers, resulting in range of shorter carbohydrate units, intermediate in complexity between the monomeric sugar and the original polymer.

## DETAILED DESCRIPTION OF THE INVENTION

The process of the present invention may be used to produce a dextrinised product from a number of different types of flour. Suitable types of flour include wheat, barley, maize, sorghum or cassava. Suitably the flour is a wheat flour.

In the process of the present invention, hydrolytic enzymes are added to aid the breakdown of glucosydic bonds of the starch polymer resulting in an oligosaccharide mixture.

The hydrolytic enzyme is an $\alpha$-amylase, more suitably the hydrolytic enzyme is a thermostable $\alpha$-amylase, such as Hitempase® available from Kerry Foods (Prince's Street, Tralee, Co. Kerry, Ireland) and Termamyl® available from Novozymes AG (Novozymes A/S, Krogshoejvej 36, 2880 Bagsvaerd, Denmark).

In one embodiment, suitably the concentration of enzyme is in the range from 0.025% to 0.5% w/w relative to flour, or from about 0.05% to about 0.5% w/w relative to the flour, more suitably the concentration of enzyme is in the range about 0.075% to about 0.5% w/w relative to flour, for example about 0.1% w/w relative to flour. In this embodiment, a particularly useful enzyme is an $\alpha$-amylase enzyme available under the trademark Termamyl® (Novozyme).

In another embodiment, suitably the concentration of enzyme is in the range from about 0.5% to 1.5% w/w relative to the flour, more suitably the concentration of enzyme is in the range about 0.7% to about 1.2% w/w relative to flour, for example about 1% w/w relative to flour In this embodiment, a particularly useful enzyme is an $\alpha$-amylase enzyme available under the trademark Hitempase®, (Kerry Foods), or available under the trademark Termamyl® (Novozyme).

Advantageously a much lower concentration of enzyme may be used in the process of the present invention in order to obtain DE values of at least 15.

Water is an important component for the gelatinisation of starch in flour. If the water content is insufficient, gelatinisation will not proceed and onward hydrolysis is not possible. The amount of water used in the process of the present invention is in the range from 30% to about 45% w/w relative to the total quantity of ingredients, for example about 36% to about 42% w/w relative to the total quantity of ingredients.

During the extrusion process, the flour entering the extruder is intensively mixed with the water as a result of the rotation of the screw elements which are designed to aid in the mixing and heating process. The use of a twin-screw extruder imparts mechanical energy and effects rapid heating. This is achieved through the speed of rotation of the screw elements within the extruder. The screw speed is about 300 to about 800 RPM, more suitably from about 450 to about 650 RPM, for example from about 500 to about 600 RPM.

Advantageously, the relatively high screw (shear) speed enables the flour and water to be thoroughly mixed and allows for the rapid throughput of said flour-water mixture through the extruder.

The operating temperature of the extruder must be such that adequate gelatinisation of the starch can take place followed by hydrolysis of said starch without inactivating the enzyme. The operating temperature of the extruder is in the range from about 80°C to about 160°C, suitably from about 90°C to about 150°C, for example about 100°C to about 140°C.

The extruder may be operated in such a way that there are zones whereby in the initial zone the conditions are optimised for process of gelatinisation and the subsequent zones optimised for the process of hydrolysis.

In one example of the process of the present invention the extruder may have one or more zones operated at different operating temperatures in which, for example, the temperature is relatively high in a first zone in which gelatinisation takes place, (the gelatinisation zone temperature), such as between about 100 to about 160°C to enable the starch granules to be disrupted, thus liberating the starch polymers from within, and relatively lower temperature in a second hydrolysis zone, (the hydrolysis zone temperature), such as between about 90°C to about 120°C to provide optimal conditions for the cleavage of glycosidic bonds whilst ensuring that the enzyme is not deactivated. Alternatively the process of the present invention may be carried out such that the average operating temperature across the length of the extruder is in the range from about 80 to about 160°C.

The time that the flour-water-enzyme mixture takes to pass through the extruder is important to enable rapid throughput. The residence time of the flour-water-enzyme mixture in the extruder is related to the screw speed, the design, length and diameter of the screw. The residence time in the extruder is from 60 to 120 seconds.

Suitable extruders for use in the process of the present invention may have the following dimensions: Screw diameter of about 20mm to about 300mm and a screw length:diameter ratio of about 30:1 to about 80:1, for example about 40:1 to about 60:1. Examples of commercially available twin-screw extruders that may be used in the process of the present invention are available from Leistritz GmbH, such as the ZSE-27 MaXX, ZSE-50 MaXX or ZSE-160 MaXX, the ZSK series of extruders from Coperion GmbH, Germany and the Omega series of extruders available from Steer India.

The degree of dextrinisation may be determined as the Dextrose Equivalent (DE). The DE value, as herein before defined above, is an industry standard way of expressing the total concentration of reducing sugars and is expressed relative to D-glucose on a dry weight basis.

The DE value may be determined by titration using the Lane/Eynon method (ISO 5377:1981 "Starch hydrolysis products - Determination of reducing power and dextrose equivalent - Lane and Eynon constant titre method").

The process of the present invention results in a dextrinised product having a DE of at least 15. Suitably the DE value is between about 15 to about 75. More suitably between about 15 to about 50, for example between about 20 to about 40 or between about 20 to about 30. Preferably the DE value is about 20.

In one embodiment there is provided a process for gelatinisation and the enzymatic hydrolysis of flour which comprises passing said flour through a twin-screw extruder in the presence of about 0.1% w/w relative to flour of a hydrolytic enzyme and in the presence of water at a moisture level of about 37% w/w relative to the total quantity of ingredients, at a screw speed of about 600 RPM and a gelatinisation zone temperature in the range about 120°C to about 140°C and a hydrolysis zone temperature in the range about 100°C to about 120°C; the dimensions of the extruder being such that the residence time of the flour through the extruder is about 60 to about 120 seconds resulting in a dextrinised product having a dextrose equivalent of about 20. Suitably the residence time is about 80 to about 100 seconds.

In another embodiment there is provided a process for gelatinisation and the enzymatic hydrolysis of flour which comprises passing said flour through a twin-screw extruder in the presence of about 0.2% w/w relative to flour of a hydrolytic enzyme and in the presence of water at a moisture level of 37% w/w relative to the total quantity of ingredients, at a screw speed of about 600 RPM and a gelatinisation zone temperature in the range about 120°C to about 140°C and a hydrolysis zone temperature in the range about 100°C to about 120°C; the dimensions of the extruder being such that the residence time of the flour through the extruder is about 60 to about 120 seconds resulting in a dextrinised product having a dextrose equivalent of about 21. Suitably the residence time is about 80 to about 100 seconds.

In another embodiment there is provided a process for gelatinisation and the enzymatic hydrolysis of flour which comprises passing said flour through a twin-screw extruder in the presence of about 1% w/w relative to flour of a hydrolytic enzyme and in the presence of water at a moisture level of about 37% w/w relative to the total quantity of ingredients, at a screw speed of about 500 RPM and a gelatinisation zone temperature in the range about 120°C to about 140°C and a hydrolysis zone temperature in the range about 100°C to about 120°C; the dimensions of the extruder being such that the residence time of the flour through the extruder is about 60 to about 120 seconds resulting in a dextrinised product having a dextrose equivalent of about 20. Suitably the residence time is about 80 to about 100 seconds.

In another embodiment there is provided a process for gelatinisation and the enzymatic hydrolysis of flour which comprises passing said flour through a twin-screw extruder in the presence of about 1% w/w relative to flour of a hydrolytic enzyme and in the presence of water at a moisture level of 41% w/w relative to the total quantity of ingredients, at a screw speed of about 600 RPM and a gelatinisation zone temperature in the range about 120°C to about 140°C and a hydrolysis zone temperature in the range about 100°C to about 120°C; the dimensions of the extruder being such that the residence time of the flour through the extruder is about 60 to about 120 seconds resulting in a dextrinised product having a dextrose equivalent of about 20. Suitably the residence time is about 80 to about 100 seconds.

Optionally, the dextrinised product may be dried. Conventional methods of drying include tray drying, vacuum band drying, spray drying, freeze drying or any such similar method.

In another aspect of the present invention there is provided a process for drying a dextrinised product which comprises passing the said dextrinised product through a twin-screw extruder having at least one vent to allow the moisture that is released in the form of water vapour to be removed. The dextrinised product to be dried may be prepared according to the process for gelatinisation and the enzymatic hydrolysis of flour as hereinbefore described.

The extruder is operated at a screw speed of 300 RPM to about 800 RPM.

Suitably about 300 to about 700 RPM, for example about 500 RPM or about 600 RPM.

Suitably the extruder may be operated at a temperature in the range of 100°C to about 140°C.

The extruder comprises at least 1 vent, more suitably there may be between 2 and 5 vents. Optionally the at least one vent through which the water vapour is released may be operated under vacuum to provide enhanced devolatilisation. Suitably, when the atmospheric pressure is 101 kPa abs, the vacuum is applied to give absolute vent pressures in the range from about 96 kPa abs to about 21 kPa abs, suitably about 86 kPa abs to about 41 kPa abs, more suitably from about 81 kPa abs to about 51 kPa abs. Alternatively, the vaccum level (kPaG) applied is from about 10 kPaG to about 80 kPaG, suitably about 15 kPaG to about 60 kPaG, more suitably from about for example about 20 kPaG to about 50kPaG. The vacuum level or absolute vent pressures may be independently adjusted for each vent.

For the avoidance of doubt, the absolute vent pressure is determined by subtracting the vaccum level (kPa G), from atmospheric pressure. For example a vaccum level of 20 kPa G results an absolute vent pressure of 81 kPa abs, when the atmospheric pressure is 101kPa abs.

During the process of drying a dextrinised product optionally other functional ingredients, such as flavours, vitamins and minerals may be added to the dextrinate. These ingredients may be added to provide functional, sensorial and / or nutritional advantages to the resulting dried extrudate.

In one embodiment of the process for drying a dextrinised product the extruder is operated at a screw speed of about 600 RPM, a temperature of about 140°C and at an absolute vent pressure of about 81 kPa abs.

In another embodiment of the process for drying a dextrinised product the extruder is operated at a screw speed of about 500 RPM, a temperature of about 140°C and at an absolute vent pressure of about 51 kPa abs.

Suitable extruders that maybe used for the process for drying a dextrinised product may have the following dimensions: Screw diameter of about 20mm to about 300mm and a screw length:diameter ratio of about 30:1 to about 80:1, for example about 40:1 to about 60:1. Examples of commercially available twin-screw extruders that may be used in the process of the present invention are available from Leistritz GmbH, such as the ZSE-27 MaXX, ZSE-50 MaXX or ZSE-160 MaXX, the ZSK series of extruders from Coperion GmbH, Germany and the Omega series of extruders available from Steer India.

## Examples

### Example 1. Production of a Dextrinised Product from Wheat Flour.

### Method

Wheat flour was fed into an Omega 30 twin-screw extruder (Steer India), at a rate of 12.8 kg/h. Flour is fed into the first zone of the extruder followed immediately by the addition of a Water/Enzyme mix.

The parameters of the extruder were set as shown in table 1.

**Table 1.** Extruder Parameters for Gelatinisation and Enzymatic Hydrolysis in an Extruder.

| Parameters | Amount/Rate | Amount/Rate |
|---|---|---|
| Enzyme (% w/w relative to flour) | 1.0 -1.5% | 0.1% - 0.5% |

(continued)

| Parameters | Amount/Rate | Amount/Rate |
|---|---|---|
| Moisture content (% w/w relative to the total quantity of ingredients | 35.0% - 42.0% | 37% |
| Screw speed (RPM) | 500 - 600 | 500 - 600 |
| Temperature °C Gelatinisation | 120-140 | 120-140 |
| Temperature °C Hydrolysis | 100-120 | 100-120 |

The extruder was optimised such that conditions were set for optimal gelatinisation in the initial zones followed by conditions conducive to enzymatic hydrolysis in the subsequent zones.

The resulting dextrinised product was anaylsed for the Dextrose Equivalent (DE), using a modification of the Lane/Eynon method as described below.

The presence of a higher protein fraction when wheat flour is used as the source of starch requires the use of a modified Lane/Eynon titration. The dextrinate cannot be fully solubilised in hot water for the standard Lane/Eynon volumetric titration due to the presence of a sparingly soluble fraction (predominantly composed of the wheat proteins). Instead, the wheat flour dextrinate is blended with hot water and gravimetrically titrated with mixed Fehling's solution in a modified Lane/Eynon gravimetric titration. This avoids issues with the suspended solids blocking up the burette normally used in the standard volumetric titration.

**Dextrose Equivalent (DE) Determination**

The DE value for dextrinised product formed from wheat flour was determined using a modified Lane/Eynon titration as follows:

**1.1. Standard glucose Solution Preparation**

A standard glucose solution was prepared by weighing 1.25 g (to the nearest 0.1mg), of dried anhydrous D-glucose and dissolving with 250 g of distilled water.

**1.2. Standardisation of Fehling's Solution**

Mixed Fehling's solution was freshly prepared and standardised daily.

Equal volumes of Fehling's A and B were mixed together. Enough Fehling's solution was mixed to cover several titrations. Each new mix of Fehling's solution must be standardised. 28.5 g of the mixed Fehling's was added to a conical flask set on a weighing scale, the scale was tared and 20 g of water was added with a few anti-bumping granules. The flask was placed on a hot plate and the contents brought to its boiling point and reduced to a simmer.

Three titrations were used for standardisation of the mixed Fehling's solution. An initial rapid titration where the methylene blue indicator was added at the start was performed to determine the approximate titration end point. This was followed by two accurate titrations where the final quantity (approximately 1 g) of standard glucose solution was added slowly.

The standard glucose solution was added into a 100 mL container on a weighing scale with a pipette and set to zero. Three drops of methylene blue indicator were added to the simmering Fehling's solution. Using the pipette, standard glucose solution was added to the Fehling's solution until the blue colour disappeared. The mass (in grams) of standard glucose required to reach the end point was noted. This mass was used as a guide for the two accurate titrations.

To perform the accurate titrations: The scale was reset to zero. Using the pipette, standard glucose solution was added to the Fehling's solution until the amount of standard glucose was 1 g short of the end point (determined by the initial titration). Three drops of methylene blue indicator were added to the mixture.

Using the pipette, the remaining standard glucose solution was added 2 to 3 drops at a time at about 10 second intervals, without interrupting the boiling, until the blue colour disappeared. This was the titration end point. The mass (in grams) of standard glucose required to reach the end point was noted. This accurate titration was repeated twice.

**1.3 Dextrinised Product Sample Solution Preparation**

The dextrinised product was weighed out accurately into a suitable container. The weight of dextrinised sample used was determined using the following equation:

$$\text{Approximate Sample Mass (g)} = 12500/(\text{Expected DE} \times \text{\% dry solids in sample})$$

250 g of boiling water was added to the dextrinate and mixed using a hand-held blender until dispersed. Approximately 50 mL of the dispersed mixture was added to a 100 mL container set on a weighing scale, with a pipette and set to zero.

Titration of the dextrinised product sample solution was carried out as described above in 1.2, using the dextrinised sample solution in place of the standard glucose solution until the end point was detected. The colour change was from blue to red. The titration was repeated twice.

Dextrinised product sample solution should be prepared and tested as soon as possible, (within 10 minutes), to minimise further hydrolysis reaction occurring.

## 1.4 Sample moisture analysis

The amount of moisture in the dextrinised product was determined by heating 5 g of dextrinised product in a hot air oven for 3 hours at 105°C and calculating the moisture content from the loss on drying.

## 1.5 DE Calculation

Taking the average mass in grams (to one decimal place), of the two titration results, the DE was determined using the following equation:

$$DE = (100 \times 10 \times a \times g \times 1000) / (b \times s \times d \times w)$$

a = Mass (in g) of D-glucose standard solution used in standardisation step

g = Mass (in g) of anhydrous D-glucose in standard glucose solution

b = Mass (in g) of the dextrinised product sample solution required to reach the titration end point

s = Mass (in g) of dextrinised product sample per 100 g in the dextrinised product sample solution

d = % dry solids in dextrinised product sample (i.e. 100% - moisture content determined by loss on drying in the oven)

w = Total mass (in g) of standard glucose solution (anhydrous D-glucose + water)

**Results** - Dextrose Equivalent of Dextrinised Product.

**Table 2.** Dextrose Equivalent values for Extruded Dextrinate Product.

| Parameters | Run 1 | Run 2 | Run 3 | Run 4 |
|---|---|---|---|---|
| Enzyme (% w/w relative to flour) | 1.0 | 1.0 | 1.4 | 1.0 |
| Moisture content (% w/w relative to the total quantity of ingredients) | 37.0 | 41.5 | 41.5 | 37.0 |
| Screw speed (RPM) | 500 | 600 | 600 | 600 |
| Temperature °C Gelatinisation | 120-140 | 120-140 | 120-140 | 120-140 |
| Temperature °C Hydrolysis | 100-120 | 100-120 | 100-120 | 100-120 |
| Residence time (s) | 90 | 87 | 87 | 80 |
| DE | 20 | 20 | 22 | 21 |

| Parameters | Run 5 | Run 6 | Run 7 | Run 8 |
|---|---|---|---|---|
| Enzyme (% w/w relative to flour) | 0.1 | 0.2 | 0.3 | 0.4 |
| Moisture content (% w/w relative to the total quantity of ingredients) | 37.0 | 37.0 | 37.0 | 37.0 |
| Screw speed (RPM) | 600 | 600 | 600 | 600 |
| Temperature °C Gelatinisation | 120-140 | 120-140 | 120-140 | 120-140 |
| Temperature °C Hydrolysis | 100-120 | 100-120 | 100-120 | 100-120 |
| Residence time (s) | Ca. 85* | Ca. 85* | Ca. 85* | Ca. 85* |
| DE | 20 | 21 | 22 | 22 |

*Not measured but estimated based on runs 1 to 4.

The results demonstrate that a dextrinised product with moderate DE values maybe obtained in an efficient manner.

**Example 2. Drying of a Dextrinised Product Following Gelatinisation and Hydrolysis. Method**

Dextrinised products from wheat flour obtained through the process of gelatinisation and hydrolysis in a first extruder (for example as described in Example 1) were fed through a second twin-screw extruder (Omega 30, Steer India), with the operating parameters set as shown in table 3.

**Table 3.** Extruder Operating Parameters for Drying and Addition of other Functional Ingredients.

| Parameters | Run 1 | Run 2 | Run 6 |
|---|---|---|---|
| Input Moisture (% w/w relative to the total quantity of ingredients) | 36 | 40 | 37 |
| Additional functional ingredients added | Yes | No | Yes |
| RPM | 500 | 600 | 500 |
| Temperature °C | 140 | 140 | 140 |
| Vent Pressure (kPa abs) | 51 | 81 | 86 |
| Vacuum Level (kPaG) | 50 | 20 | 15 |

The dextrinised extrudate was pumped into the second extruder directly from the first extruder, by means of a coupling pipe.

The moisture level of the dried product was determined by measuring the loss in weight upon drying in an oven at 105°C for 3 hours and calculating the moisture content from the loss on drying.

**Results.**

**Table 4.** Output moisture Levels of Dextrinised Product dried in an extruder.

| Parameters | Run 1 | Run 2 | Run 6 |
|---|---|---|---|
| Input Moisture(% w/w relative to the total quantity of ingredients) | 36 | 40 | 37 |
| Output Moisture (% w/w of sample) | 8 | 2 | 4 |

The results demonstrate that an extruder may be used to efficiently dry a dextrinised product.

**Claims**

1. A process for gelatinisation and the enzymatic hydrolysis of flour which comprises passing said flour through a twin-screw extruder in the presence of 0.025 % to 1.5 % w/w relative to flour of an $\alpha$-amylase hydrolytic enzyme and in the presence of water at a moisture level from 30% to 45% w/w relative to the total quantity of ingredients, at a screw speed from 300 RPM to 800 RPM and a temperature in the range from 80°C to 160°C; the dimensions of the extruder being such that the residence time of the flour through the extruder is from 60 to 120 seconds and results in a dextrinised product having a dextrose equivalent of at least 15.

2. A process as claimed in claim 1 wherein the enzyme is present in an amount in the range from 0.075 % to 0.5 % w/w relative to the flour.

3. A process as claimed in claim 1 wherein the enzyme is present in an amount of 0.7% to 1.2 % w/w relative to the flour.

4. A process as claimed in any one claims 1 to 3 wherein the water is present in the range from 36% to 42% w/w relative to the total quantity of ingredients.

5. A process as claimed in any one of claims 1 to 4 wherein the screw speed is 450 to 650 RPM.

6. A process as claimed in any one of claims 1 to 5 wherein the temperature is in the range from 90°C to 150°C.

7. A process as claimed in claim 2, which comprises passing said flour through a twin-screw extruder in the presence of 0.1% w/w relative to flour of an $\alpha$-amylase hydrolytic enzyme and in the presence of water at a moisture level of 37% w/w relative to the total quantity of ingredients, at a screw speed of 600 RPM and a gelatinisation zone temperature in the range 120°C to 140°C and a hydrolysis zone temperature in the range 100°C to 120°C; the dimensions of the extruder being such that the residence time of the flour through the extruder is 60 to 120 seconds resulting in a dextrinised product having a dextrose equivalent of 20.

8. A process as claimed in claim 3 which comprises passing said flour through a twin-screw extruder in the presence of 1% w/w relative to flour of an $\alpha$-amylase hydrolytic enzyme and in the presence of water at a moisture level of 37% w/w relative to the total quantity of ingredients, at a screw speed of 500 RPM and a gelatinisation zone temperature in the range 120°C to 140°C and a hydrolysis zone temperature in the range 100°C to 120°C; the dimensions of the extruder being such that the residence time of the flour through the extruder is 60 to 120 seconds resulting in a dextrinised product having a dextrose equivalent of about 20.

9. A process as claimed in claim 3 which comprises passing said flour through a twin-screw extruder in the presence of 1% w/w relative to flour of an $\alpha$-amylase hydrolytic enzyme and in the presence of water at a moisture level of 41% w/w relative to the total quantity of ingredients, at a screw speed of 600 RPM and a gelatinisation zone temperature in the range 120°C to 140°C and a hydrolysis zone temperature in the range 100°C to 120°C; the dimensions of the extruder being such that the residence time of the flour through the extruder is 60 to 120 seconds resulting in a dextrinised product having a dextrose equivalent of 20.

10. A process as claimed in any one of claims 1 to 9 wherein the dextrinised product is dried by passing it through a twin-screw extruder having at least one vent to allow the moisture that is released in the form of water vapour to be removed and wherein the extruder is operated at a screw speed in the range 300 RPM to 700 RPM.

11. A process as claimed in claim 10 wherein the extruder is operated at a temperature in the range of 70°C to 180°C.

12. A process as claimed in claim 10 or 11 wherein the at least 1 vent is operated at an absolute vent pressure in the range from 41 kPa abs to 96 kPa abs.

13. A process as claimed in any one of claim 10 to 12 wherein the extruder is operated at a screw speed of 500 RPM, a temperature of 140°C and at an absolute vent pressure of 51 kPa abs.

14. A process as claimed in any one of claim 10 to 12 wherein the extruder is operated at a screw speed of 600 RPM, a temperature of about 140°C and at an absolute vent pressure of 81 kPa abs.

**Patentansprüche**

1. Verfahren zur Gelatinierung und enzymatischen Hydrolyse von Mehl, umfassend das Durchleiten des Mehls durch einen Doppelschneckenextruder in Anwesenheit von 0,025% bis 1,5% Gew./Gew., relativ zu Mehl, eines hydrolytischen $\alpha$-Amylase-Enzyms und in Anwesenheit von Wasser bei einem Feuchtigkeitsgehalt von 30% bis 45% Gew./Gew., relativ zu der Gesamtmenge der Bestandteile, bei einer Schneckendrehzahl von 300 U/min bis 800 U/min und einer Temperatur im Bereich von 80 °C bis 160 °C; wobei die Abmessungen des Extruders so sind, dass die Verweilzeit des Mehls durch den Extruder 60 bis 120 Sekunden beträgt und zu einem dextrinierten Produkt mit einem Dextroseäquivalent von mindestens 15 führt.

2. Verfahren wie in Anspruch 1 beansprucht, wobei das Enzym in einer Menge im Bereich von 0,075% bis 0,5% Gew./Gew. relativ zum Mehl vorliegt.

3. Verfahren wie in Anspruch 1 beansprucht, wobei das Enzym in einer Menge von 0,7% bis 1,2% Gew./Gew. relativ zum Mehl vorliegt.

4. Verfahren wie in irgendeinem der Ansprüche 1 bis 3 beansprucht, wobei das Wasser im Bereich von 36% bis 42% Gew./Gew. relativ zur Gesamtmenge der Bestandteile vorliegt.

5. Verfahren wie in irgendeinem der Ansprüche 1 bis 4 beansprucht, wobei die Schneckendrehzahl 450 bis 650 U/min beträgt.

6. Verfahren wie in irgendeinem der Ansprüche 1 bis 5 beansprucht, wobei die Temperatur im Bereich von 90 °C bis 150 °C liegt.

**7.** Verfahren wie in Anspruch 2 beansprucht, umfassend das Durchleiten des Mehls durch einen Doppelschneckenextruder in Anwesenheitvon 0,1% Gew./Gew., relativ zu Mehl, eines hydrolytischen $\alpha$-Amylase-Enzyms und in Anwesenheitvon Wasser bei einem Feuchtigkeitsgehalt von 37% Gew./Gew., relativ zu der Gesamtmenge der Bestandteile, bei einer Schneckendrehzahl von 600 U/min und einer Gelatinierungszonentemperatur im Bereich von 120 °C bis 140 °C und einer Hydrolysezonentemperatur im Bereich von 100 °C bis 120 °C; wobei die Abmessungen des Extruders so sind, dass die Verweilzeit des Mehls durch den Extruder60 bis 120 Sekunden beträgt, was zu einem dextrinierten Produkt mit einem Dextroseäquivalent von 20 führt.

**8.** Verfahren wie in Anspruch 3 beansprucht, umfassend das Durchleiten des Mehls durch einen Doppelschneckenextruder in Anwesenheitvon 1% Gew./Gew., relativ zu Mehl, eines hydrolytischen $\alpha$-Amylase-Enzyms und in Anwesenheitvon Wasser bei einem Feuchtigkeitsgehalt von 37% Gew./Gew., relativ zu der Gesamtmenge der Bestandteile, bei einer Schneckendrehzahl von 500 U/min und einer Gelatinierungszonentemperatur im Bereich von 120 °C bis 140 °C und einer Hydrolysezonentemperatur im Bereich von 100 °C bis 120 °C; wobei die Abmessungen des Extruders so sind, dass die Verweilzeit des Mehls durch den Extruder 60 bis 120 Sekunden beträgt, was zu einem dextrinierten Produkt mit einem Dextroseäquivalent von etwa 20 führt.

**9.** Verfahren wie in Anspruch 3 beansprucht, umfassend das Durchleiten des Mehls durch einen Doppelschneckenextruder in Anwesenheitvon 1% Gew./Gew., relativ zu Mehl, eines hydrolytischen $\alpha$-Amylase-Enzyms und in Anwesenheitvon Wasser bei einem Feuchtigkeitsgehalt von 41% Gew./Gew., relativ zu der Gesamtmenge der Bestandteile, bei einer Schneckendrehzahl von 600 U/min und einer Gelatinierungszonentemperatur im Bereich von 120 °C bis 140 °C und einer Hydrolysezonentemperatur im Bereich von 100 °C bis 120 °C; wobei die Abmessungen des Extruders so sind, dass die Verweilzeit des Mehls durch den Extruder 60 bis 120 Sekunden beträgt, was zu einem dextrinierten Produkt mit einem Dextroseäquivalent von 20 führt.

**10.** Verfahren wie in irgendeinem der Ansprüche 1 bis 9 beansprucht, wobei das dextrinierte Produkt durch Hindurchleiten durch einen Doppelschneckenextruder getrocknet wird, der mindestens eine Entlüftung aufweist, um die Entfernung der in Form von Wasserdampf freigesetzten Feuchtigkeit zu ermöglichen, und wobei der Extruder bei einer Schneckendrehzahl im Bereich von 300 U/min bis 700 U/min betrieben wird.

**11.** Verfahren wie in Anspruch 10 beansprucht, wobei der Extruder bei einer Temperatur im Bereich von 70 °C bis 180 °C betrieben wird.

**12.** Verfahren wie in Anspruch 10 oder 11 beansprucht, wobei die mindestens 1 Entlüftung bei einem absoluten Entlüftungsdruck im Bereich von 41 kPa abs bis 96 kPa abs betrieben wird.

**13.** Verfahren wie in irgendeinem der Ansprüche 10 bis 12 beansprucht, wobei der Extruder bei einer Schneckendrehzahl von 500 U/min, einer Temperatur von 140 °C und einem absoluten Entlüftungsdruckvon 51 kPa abs betrieben wird.

**14.** Verfahren wie in irgendeinem der Ansprüche 10 bis 12 beansprucht, wobei der Extruder bei einer Schneckendrehzahl von 600 U/min, einer Temperatur von etwa 140 °C und einem absoluten Entlüftungsdruckvon 81 kPa abs betrieben wird.

**Revendications**

**1.** Procédé pour gélatinisation et l'hydrolyse enzymatique de farine qui comprend le passage de ladite farine à travers une extrudeuse à double vis en présence de 0,025 % à 1,5 % masse/masse par rapport à la farine d'une enzyme hydrolytique d'a-amylase et en présence d'eau à une teneur en humidité de 30 % à 45 % masse/masse par rapport à la quantité totale d'ingrédients, à une vitesse de vis de 300 tr/min à 800 tr/min et une température dans l'intervalle de 80°C à 160°C ; les dimensions de l'extrudeuse étant telles que le temps de séjour de la farine à travers l'extrudeuse est de 60 à 120 secondes et résulte en un produit dextrinisé ayant un équivalent dextrose d'au moins 15.

**2.** Procédé selon la revendication 1, dans lequel l'enzyme est présente dans une quantité dans l'intervalle de 0,075 % à 0,5 % masse/masse par rapport à la farine.

**3.** Procédé selon la revendication 1, dans lequel l'enzyme est présente dans une quantité de 0,7 % à 1,2 % masse/masse par rapport à la farine.

4. Procédé selon l'une quelconque des revendications 1 à 3, dans lequel l'eau est présente dans l'intervalle de 36 % à 42 % masse/masse par rapport à la quantité totale d'ingrédients.

5. Procédé selon l'une quelconque des revendications 1 à 4, dans lequel la vitesse de vis est de 450 à 650 tr/min.

6. Procédé selon l'une quelconque des revendications 1 à 5, dans lequel la température se trouve dans l'intervalle de 90°C à 150°C.

7. Procédé selon la revendication 2, qui comprend le passage de ladite farine à travers une extrudeuse à double vis en présence de 0,1 % masse/masse par rapport à la farine d'une enzyme hydrolytique d'a-amylase et en présence d'eau à une teneur en humidité de 37 % masse/masse par rapport à la quantité totale d'ingrédients, à une vitesse de vis de 600 tr/min et une température de zone de gélatinisation dans l'intervalle de 120°C à 140°C et une température de zone d'hydrolyse dans l'intervalle de 100°C à 120°C ; les dimensions de l'extrudeuse étant telles que le temps de séjour de la farine à travers l'extrudeuse est de 60 à 120 secondes résultant en un produit dextrinisé ayant un équivalent dextrose de 20.

8. Procédé selon la revendication 3, qui comprend le passage de ladite farine à travers une extrudeuse à double vis en présence de 1 % masse/masse par rapport à la farine d'une enzyme hydrolytique d'a-amylase et en présence d'eau à une teneur en humidité de 37 % masse/masse par rapport à la quantité totale d'ingrédients, à une vitesse de vis de 500 tr/min et une température de zone de gélatinisation dans l'intervalle de 120°C à 140°C et une température de zone d'hydrolyse dans l'intervalle de 100°C à 120°C ; les dimensions de l'extrudeuse étant telles que le temps de séjour de la farine à travers l'extrudeuse est de 60 à 120 secondes résultant en un produit dextrinisé ayant un équivalent dextrose d'environ 20.

9. Procédé selon la revendication 3, qui comprend le passage de ladite farine à travers une extrudeuse à double vis en présence de 1 % masse/masse par rapport à la farine d'une enzyme hydrolytique d'a-amylase et en présence d'eau à une teneur en humidité de 41 % masse/masse par rapport à la quantité totale d'ingrédients, à une vitesse de vis de 600 tr/min et une température de zone de gélatinisation dans l'intervalle de 120°C à 140°C et une température de zone d'hydrolyse dans l'intervalle de 100°C à 120°C ; les dimensions de l'extrudeuse étant telles que le temps de séjour de la farine à travers l'extrudeuse est de 60 à 120 secondes résultant en un produit dextrinisé ayant un équivalent dextrose de 20.

10. Procédé selon l'une quelconque des revendications 1 à 9, dans lequel le produit dextrinisé est séché en le faisant passer à travers une extrudeuse à double vis ayant au moins une mise à l'air pour permettre à l'humidité qui est libérée dans la forme de vapeur d'eau d'être éliminée et dans lequel l'extrudeuse fonctionne à une vitesse de vis dans l'intervalle de 300 tr/min à 700 tr/min.

11. Procédé selon la revendication 10, dans lequel l'extrudeuse fonctionne à une température dans l'intervalle de 70°C à 180°C.

12. Procédé selon la revendication 10 ou 11, dans lequel la au moins 1 mise à l'air fonctionne à une pression absolue de mise à l'air dans l'intervalle de 41 kPa abs à 96 kPa abs.

13. Procédé selon l'une quelconque des revendications 10 à 12, dans lequel l'extrudeuse fonctionne à une vitesse de vis de 500 tr/min, une température de 140°C et une pression absolue de mise à l'air de 51 kPa abs.

14. Procédé selon l'une quelconque des revendications 10 à 12, dans lequel l'extrudeuse fonctionne à une vitesse de vis de 600 tr/min, une température d'environ 140°C et une pression absolue de mise à l'air de 81 kPa abs.

FIG. 1

EP 3 286 306 B1

*FIG. 2*

EP 3 286 306 B1

**FIG. 3**

EP 3 286 306 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62150970 B

- US 62241314 B

**Non-patent literature cited in the description**

- **THAVA VASANTHAN ; JUDY YEUNG ; RATNA-JOTHI HOOVER.** Dextrinisation of Starch in Barley Flours with Thermostable α-amylase by Extrusion Cooking. *Starch/Stärke,* 2001, vol. 53, 616-622

- **GOVINDASAMY et al.** *Carbohydrate Polymers,* 1997, vol. 32, 267-274
- **ROUSSEL et al.** *Lebensm.-Wiss. U.- Technol.,* 1991, vol. 24, 449-458
- **VASANTHAN et al.** *Starch/Stärke,* 2001, vol. 53, 616-622